# EUROPEAN PATENT SPECIFICATION

(11) **EP 0 632 813 B1**
(45) Date of publication and mention of the grant of the patent: **15.12.1999**
(21) Application number: 93903950.9
(22) Date of filing: 11.02.1993
(51) Int. Cl.: C07H 7/02, C08B 37/02, C07K 2/00, A61K 31/70, A61K 31/73

(54) **AMADORI REACTION COMPOUNDS AND PRODUCTS, PROCESS FOR THEIR MANUFACTURE, AND THEIR USE**
AMADORI-REAKTION-VERBINDUNGEN, UND -PRODUKTE, VERFAHREN ZU DEREN HERSTELLUNG UND DEREN VERWENDUNG
PRODUITS ET COMPOSES DE TRANSPOSITION D'AMADORI, LEUR PROCEDE DE FABRICATION ET LEUR UTILISATION

(30) Priority: 13.02.1992 PL 293464; 03.03.1992 EP 92103614
(43) Date of publication of application: 11.01.1995
(73) Proprietor: Torf Establishment, FL-9490 Vaduz (LI)
(72) Inventor: MIODUSZEWSKI, Jan, Zbigniew, 01-057 Warszawa (PL); WITKIEWICZ, Krystyna, 53-125 Wroclaw (PL); INGLOT, Anna, 53-651 Wroclaw (PL)
(74) Representative: Büchel, Kurt F., Dr.
(86) International application number: EP9300327
(87) International publication number: WO9316087

(56) References cited:
- EP-A- 0 111 211
- EP-A- 0 152 856
- WO-A-89/09786
- WO-A-92/16216
- CA-A- 673 730
- DE-A- 3 601 472
- US-A- 4 022 920
- CARBOHYDRATE RESEARCH. vol. 92, 1981, AMSTERDAM NL pages 37 - 49 ALTENA H.J. ET AL 'Analysis of the 220-MHz, P.M.R. spectra of some products of the Amadori and Heyns rearrangements' See Scheme 1
- ALFRED GOTTSCHALK 'B.B.A. Library, Volume 5, Glycoproteins - Their Composition, Structure and Function - Part A' 1972 , ELSEVIER PUBLISHING COMPANY , AMSTERDAM
- JUSTUS LIEBIGS ANNALEN DER CHEMIE vol. 703, 1967, WEINHEIM DE pages 202 - 214 HEYNS K. ET AL 'Quantitative Untersuchungen der Reaktion von Hexosen mit Amins{uren'

## Description

The present invention relates to a novel use of Amadori rearrangement compounds and of products having at least partly entered an Amadori rearrangement as per the following reaction scheme, to the production thereof as well as to specific mixtures of such compounds and to pharmaceutical and cosmetic compositions containing these mixturesproducts: 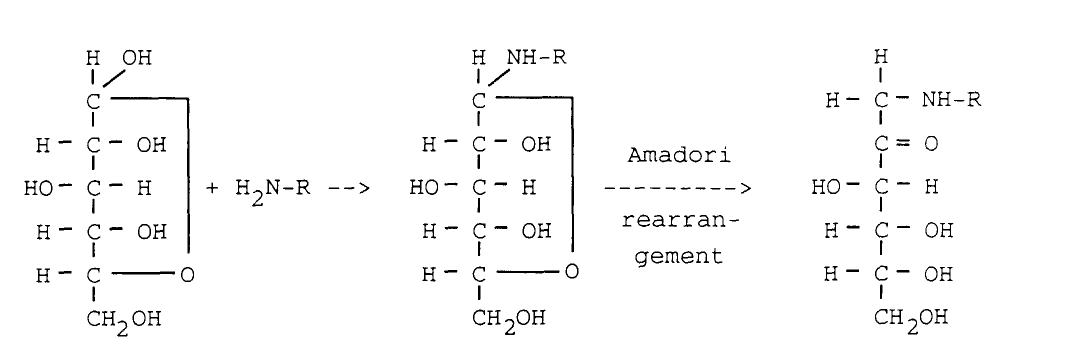 Amadori rearrangement compounds are known; they are reaction products, for example, of an amino acid or a peptide with a sugar, oligo- or polysaccharide having entered an Amadori rearrangement (Henri Borsook et al.,J. Biol. Soc. 215 (1955),111-124, ). Thus, in DE-C-3914354, a water-soluble glycoprotein of an amino acid and a sugar is described which is isolated from an extract of Avena sativa seeds. Further, EP-A-406087 describes water-soluble polysaccharide-glycopeptide complexes which are derived from the cell wall of Gram positive bacterium, and J. Biol. Chem. 1985, 260/9 states that NMR-spectroscopy has been used to characterize Amadori rearrangement compounds formed by reaction of glucose with free amino groups of protein.

EP-A-152 856 reports the chemical synthesis of biologically active N-acylated 1-alkylamino-1-deoxy-ketose analogues wherein the final products are obtained by reacting a sugar with an alkylamino compound and carrying out an Amadori rearrangement to yield a 1-alkylamino-1-deoxy-ketose analogue, followed by a final acylation of the amino group. The process requires at least a three step procedure for subsequently carrying out the necessary chemical reactions as well as the input of more or less expensive chemical reagents involved in the process, in addition to the basic reactants.

WO 89/09786 teaches amongst others a LHRH antagonist peptide and a calcitonin peptide modified by at least one Amadori rearranged sugar residue. The resulting products are useful for inhibiting luteinizing hormone secretion and calcium regulation in the body, respectively. It also discloses various processes for the manufacture of these products, e.g. using as the solvent a mixture of DMF/AcOH and carrying out the reaction at 55°C for 4 hours.

EP 111 211 teaches amongst others the production of immunogenic agents to induce the release of antibodies for the specific detection of compounds containing Amadori rearranged sugars in the blood of patients. The immunogenic agent can be a natural protein or polypeptide or a synthetically prepared macromolecule carrying 1-deoxy-fructosyl residues. The peptides have molecular weights of between 4,000 and 10,000,000 daltons. Also disclosed are methods of glycosylating such carrier macromolecules (i.e., peptides) comprising, for instance, exposing such a macromolecule to an excess of glucose under mild, phosphate buffered pH conditions and incubating the mixture at about 35 - 40°C for a time period of about 10 - 20 days.

The invention now describes the novel use of specific Amadori rearrangement compounds as described in claim 1. Preferred embodiments and improvements are described in claims 2 to 10 and 23. These compounds reduce potassium ferricyanide, a test reaction for biologically active substances formed in a reaction of sugar and amino acid.

Immunostimulating drugs are indeed known from natural sources such as mistletoe extracts, peat extracts etc. with the drawback of expensive treatment of large quantities of raw material to obtain a few grams of active substance and of uncontrollable impurities that might lead to toxicity and side effects and, therefore, to problems with administration during practical use due to the complex nature and the hardly reproducible composition.

In the past, nobody has tested fractions obtained from the various steps of purification (carried out in order to get rid of ballast substances and impurities) of a mistletoe or peat extract containing Amadori rearrangement compounds for biological activity so far.

Comparable products or product mixtures from artificial sources, such as interferon, or obtained by genetic engineering methods are even more expensive to make. Furthermore, the molecules of human interferon are very often too big to penetrate the human cell wall so that only a fraction of the administered dose is effectively becoming active. Also, genetic engineering products usually have side effects and some are even toxic. Furthermore, some of them act effectively on one day and not on the next day, for reasons unknown so far.

Surprisingly, it has now been found that nearly any simple amino acid/sugar complex after having at least partly entered an Amadori rearrangement does not show any of the above-mentioned disadvantages but, on the contrary, has a surprisingly high immunological activity. It can therefore be used in pharmaceutical formulations and in cosmetics. These small molecules easily penetrate the cell wall and virtually act as a nutrient. They induce the formation of natural interferon and other cytokines, including tumor necrosis factor. Even three days after the administration, they still show this stimulating effect on biological activity. This effect increases with increasing completion of the Amadori rearrangement and decreases again with increasing decomposition of the complex.

Instead of simple sugars also - preferably low molecular weight, especially of less than 1000 daltons - polysaccharides may be used, for instance dextrane, which react similarly. Polysaccharides show biological activity and may retain some of this activity after they become oligosaccharides.

Very little has been known heretofore about the biological activity of these compounds. It was now found that combinations of these substances in contact with human leukocytes produce interferon and other cytokines. This is called polyclonal activation of the cells.

It is possible to test the substances produced under the influence of these compounds and to determine the biological activities in international units relevant to specific cytokines. These compounds are of especially high biological activity within a range of pure substance concentrations from 1-100 µg/ml. Within the molecule, the specific nature of the amino acid is more important than the nature of the sugar part of the molecule.

Reacticn products of L-aspartic acid with glucose or galactose - after having gone through the Amadori rearrangement - when contacted with human leukocytes and incubated in tissue culture media at 37°C for 20 hours in an atmosphere of 5% CO₂ will produce from 30 - 1000 antiviral units of interferon. The interferon is measured in a bioassay using human cancer cells. Under the influence of these compounds, tumor necrosis compounds may also be produced.

The products which allow such an unexpected use have the general formula

R₁-NH-R₂

wherein
- R₁: represents a 1-deoxy-2-ketose radical derived from the group of simple sugars, oligo- and - preferably low molecular weight, especially of less than 1000 daltons - polysaccharides, and
- R₂: represents an amino acid or a - preferably low molecular weight, especially of less than 1000 daltons - peptide radical.

Thus, the group of biologically active compounds may cover either the specific Amadori rearrangement compounds described above or the N-substituted derivatives of a number of different amino acid compounds and one simple sugar, oligo- or - preferably low molecular weight, especially of less than 1000 daltons - polysaccharide, or N-substituted derivatives of one amino acid compound and a number of simple sugars, oligo- and/or such polysaccharides, or else any combination of such derivatives, every single one of them having sufficient biological activity.

Preferably, R₁ in the above formula may be a radical selected from the D-form of simple sugars, especially (but not exclusively) from the D-form of glucose, xylose, galactose, rhamnose, fructose, mannose, 6-deoxyglucose, glucosamine and galactosamine; R₂ may be a radical selected from the L-form of amino - acid compounds such as serine, glycine, histidine, arginine, glutamine, asparagine, alanine, aspartic acid, glutamic acid, phenylalanine, threonine, cysteine, cystine, methionine, hydroxyproline, tryptophane, proline, tyrosine, valine, isoleucine, leucine and lysine or else peptides of these amino acids in any combination.

The invention further relates to a process for obtaining the above mentioned compounds and products as described in claim 11 and whereby an intermediate is formed of the formula

R1'-NH-R2'

wherein
R1' is a 1-deoxy-2-ketose radical in a straight carbon chain or any O-bridged form of a simple sugar or an oligo- or polysaccharide, and
R2' represents an amino acid or a peptide radical,
said intermediate being at least partly subjected to an Amadori rearrangement reaction by continued heating of the reaction mixture - preferably under pressure - and simultaneously or subsequently removing the solvents. Preferred embodiments of the process are described in claims 12 to 15.

In some cases, especially when an amino acid having two carboxyl groups is used, it is advantageous to add to the process a buffer salt, such as sodium bicarbonate, preferably in a molar ratio of 1:1.

It was further found that the Amadori rearrangement compounds are relatively susceptible to decomposition, and that decomposition products have the nature of dark brown and tar-like, unidentified compounds having lost their biological activity. Therefore it is preferred to stop the Amadori rearrangement reaction at a stage at which the reaction mixture becomes light orange-brown in colour.

It is interesting to note that the intermediate reaction products formed when the originally opaque solution of the amino acid becomes clear (before the Amadori rearrangement), are easily hydrolysed, i.e. the reaction is reversible. With increasing rearrangement, the reversibility diminishes, i.e. the products become more stable, and the colour gradually changes from light yellow to light orange, and then finally to orange-brown when the Amadori rearrangement seems to be complete. Samples taken during such rearrangement reactions and tested (according to various procedures described later) proved that the biological activity increases with the Amadori rearrangement progressing, and decreases when further heating results in decomposition, for which a colour change to dark brown is a sign. Immediate reduction of ferricyanide and the resulting colour change will occur if the reaction mixture contains other keto groups and/or sulfur containing amino acids such as cysteine; otherwise it will occur within 3 to 5 min, which is a good check for the degree of Amadori rearrangement developed. Unreacted sugars would show the colour change after half an hour or several hours only.

Isolation of the pure Amadori rearrangement products is carried out according to known methods based on binding the mixture on a strong cation-exchanger (such as Amberlite^{(R)} or Dowex ^{(R)}, successively eluting with ammonia water, evaporating a chosen defined fraction of the eluate under reduced pressure and crystallising the pure compound from anhydrous methanol (J.E. Hodge and B.E. Fisher, Methods in Carbohydrate Chemistry, Vol.II,Reactions of Carbohydrates, Academic Press, N.Y., London, 1963, page 105-106; or Borsook et al. as quoted earlier; or J. Dubourg and P. Devilliers, Bull. Soc. Chim. France 1957, 333-336).

In the process according to the present invention, all the above-mentioned preferences regarding the kind of radicals derived from simple sugar and amino acid compounds remain unchanged. Additionally, a preferred mixture of sugar substrates comprises the D-forms of glucose, xylose, galactose, rhamnose and fructose in a weight ratio of about 20:10:4:1:1, while the preferred mixture of amino acid substrates comprises the L-forms of serine, glycine, histidine, arginine, alanine, proline, tyrosine, valine, leucine, isoleucine and lysine in a weight ratio of 20.5:35.8:35.8:132:180:360:216:160:72:68:780.

As already mentioned, the Amadori rearrangement compounds are able to reduce potassium ferricyanide, such a chemical test reaction providing a basis for quick determination of the biological activity of the composition formed in a reaction cf sugar and amino acid.

It has been found that Amadori rearrangement compounds are especially active if a mixture of simple sugars of the same composition and in the same weight ratio as occurring in natural peat extracts are reacted with a mixture of amino acid compounds of the same composition arc in the same weight ratio as occurring in natural peat extracts in the presence of an aqueous solvent, preferably adding a lower alcohol - and optionally inorganic trace elements occurring in such natural peat extracts - at elevated temperature (and optionally under pressure), and subsequently prolonging the heating in order to cause an Amadori rearrangement of the obtained products, simultaneously or subsequently expelling the solvents, stopping the rearrangement reaction at the point when the reaction mixture becomes light orange-brown in colour, drying the products thus obtained and purifying the same by means of column chromatography and collecting the fractions that cause maximum reduction of potassium ferricyanide.

In one embodiment, the invention uses instant pharmaceutical formulations containing as an active ingredient at least one reaction product of the formula R₁'-NH-R₂' or a specific compound of the formula R₁-NH-R₂ together with a pharmaceutically acceptable carrier and/or an adjuvant and/or optionally a lubricant in a weight ratio of active ingredient to the remaining components of between 1:1 and 1:100, preferably 1:8 to 1:20 and most preferably about 1:9.

Another advantageous pharmaceutical formulation contains - in addition to the active ingredient - lactose and a lubricant, the weight ratio of lactose to the lubricant being between 20:1 and 100:1, preferably 50:1.

These pharmaceutical preparations are used to treat and/or prevent hematological diseases and/or stimulate the immunosystem of humans and/or mammals by the induction of cytokine formation.

Another use for the active ingredients is in cosmetic preparations. The active ingredient is present in such preparations in amounts of 0,01-10% by weight, preferably 0,01-1% by weight and especially in amounts of 0,05-0,1%. These cosmetic preparations contain - besides the active ingredient - usual carriers, adjuvants, enriching components and/or fragrants.

The present invention will be further explained and demonstrated in the following examples, which do not limit in any respect the scope of the present invention.

### Example 1:

The 25 ml flask of a rotary evaporator placed in a heated water bath was charged with:
1.47 g (0.01 M) L-glutamic acid
0.84 g (0.01 M) NaHCO₃
0.91 g D-glucose
0.91 g galactose and
3.00 ml of redistilled water

The mixture was heated up to 80°C whereby - preferably under reduced pressure - 50% of the water were evaporated and then - under atmospheric pressure - heated to a temperature of 80-85°C, at which it was kept for 120 min until it became orange in colour. The syrup-like concentrated aqueous solution was then evaporated to dryness under reduced pressure. The orange-red solid reaction product thus obtained was marked with the symbol D-10 and saved for biological tests.

### Example 2:

The 25 ml flask of a rotary evaporator placed in a heated water bath was charged with:
1.33 g (0.01 M) L-aspartic acid
0.84 g (0.01 M) NaHCO₃
0.91 g of D-glucose
0.91 g of galactose and
3.00 ml of redistilled water

The mixture was heated up to 80°C with stirring (by means of rotation), concentrated under pressure, whereby a total volume of 1.5 ml water was vaporized, and then treated under atmospheric pressure at a temperature of 80-85°C until it became light orange in colour; this took place within approx. 60 min.

The reaction mixture was a syrup-like concentrated aqueous solution and was dried under reduced pressure. The resulting reaction product was a dry, yellow-orange powder. It was marked with the symbol D-11 and saved for biological tests.

### Example 3:

The 25 ml flask of a rotary evaporator placed in a heated water bath was charged with:
1.05 g (0.01 M) L-serine
0.91 g D-glucose
0.91 g galactose and
2.50 ml redistilled water

The mixture was heated to 85-92°C with stirring (by means of rotation). After 100 min, the solution became orange in colour. The pressure was reduced and the mixture was evaporated to dryness. The reaction product on the walls of the flask formed an orange transparent layer. The reaction product was scraped off and powdered. It was marked with the symbol D-12 and saved for biological tests.

### Example 4:

The 25 ml flask of a rotary evaporator placed in a heated waterbath was charged with:
0.66 g (0.005 M) of D-aspartic acid
0.42 g (0.005 M) of NaHCO₃
0.45 g of D-glucose
0.45 g of galactose and
3.00 ml of redistilled water

The mixture was heated up to 80°C, evaporated - under pressure - whereby a volume of 1.5 ml water was vaporized, and then treated - under atmospheric pressure - at a temperature of 85°C. After 60 min. heating the mixture became orange in colour; the pressure was reduced and the resulting syrup-like concentrated aqueous solution evaporated to dryness. Before the residue became definitely dry, two times 10 ml water-free ethanol were introduced into the flask and evaporated in order to eliminate residual moisture. The dry reaction product thus obtained was powdered, marked with the symbol D-13 and saved for biological tests.

### Example 5:

In order to obtain - in a synthetic way - an equivalent of the biologically active fraction of a certain natural peat extract, the flask of the rotary evaporator placed in the heated water bath was charged with:
20.5 mg L-serine
35.8 mg L-glycine
35.8 mg L-histidine
132.0 mg L-arginine
180.0 mg L-alanine
360.0 mg L-proline
216.0 mg L-tyrosine
160.0 mg L-valine
68.0 mg L-isoleucine
72.0 mg L-leucine
780.0 mg L-lysine
2000.0 mg D-glucose
1000.0 mg D-xylose
400.0 mg D-galactose
1 .0 mg D-rhamnose
100.0 mg D-fructose
6.0 ml redistilled water

The mixture was stirred by means of rotation and heated under pressure for 45 min at a temperature rising from 75°C to 86°C. During that period, approx. 3 ml of water were evaporated and the substrates were totally dissolved. The mixture was then treated for 30 min under atmospheric pressure at a temperature of 85-86°C for an Amadori rearrangement. During that period the solution quickly became red-brown in colour. The pressure was reduced, and heating at 84°C was continued, thus simultaneously evaporating the solvents. At the end of evaporation, two times 15 ml water-free ethanol were introduced and the reaction mixture was brought to dryness. The flask with the dried reaction product was placed in a desiccator over calcium chloride for 18 hours; then the reaction product was powdered. Approx. 4.5g of a powdered product were obtained and marked with the symbol EK₂-S.

A portion of 4g of this reaction product was dissolved in 20 ml of distilled water and placed on a chromatographic column of 25 mm x 330 mm size, filled with a sorbent Amberlite^{(R)} XAD-2, analytical grade. The column was eluted with 0.4 ml/min distilled water. Fractions of 10 ml volume were collected to a total volume of 450 ml. The content of the fractions was monitored chromatographically. Fractions of consecutive numbers 11-13 were combined and evaporated under reduced pressure. These fractions were characterised by a high content of Amadori rearrangement compounds (confirmed with the potassium ferricyanide reduction test). The product was saved for biological tests under the symbol of EK₂-S-11.

Biological tests for determining the biological activity were carried out with immunised Balb/C mice of both sexes, at the age of 8-10 weeks. Immunisation of mice is achieved by peritcneal administration of 0.2 ml of a 10% suspension of sheep erythrocytes (SRBC), i.e. of 6x10⁸ cells. The erythrocytes are fixed in a sterile Alsever's solution of the following composition:

| | |
|---|---|
| glucose | 2.05 g |
| sodium citrate | 0.8 g |
| sodium chloride | 0.42 g |
| citric acid | 0.055 g |
| redistilled water to | 100 ml |

Into such Alsever's solution, a sheep blood cell aseptic sample is introduced in a ratio of 1:1 and the mixture is kept for at least 3 days at +4°C. The thus stabilised erythrocytes are then sampled aseptically and introduced into a phosphate buffered salt sclution (PBS) in order to wash them out. Erythrocytes are rinsed with PBS twice, and are centrifuged for 10 min at 2000 rpm. The washed out cells are used in the form of a 10% suspension in PBS. Such a suspension is used for the immunisation of Balb/C mice.

The reaction product to be tested was administered intraperitoneally (i.p.) or orally (p.o.) four times at chosen doses, the first administration taking place 2 hours before immunisation of the mouse with SRBC, while the remaining three dosages were administered after immunisation at 24 h intervals.

Each tested group of animals was treated with different doses of the tested reaction product: 10 mg/kg, 1 mg/kg, 0.1 mg/kg and 0.01 mg/kg. A control group of animals was also immunised with SRBC, but instead of the substance to be tested, 0.2 ml of PBS were administered at the same time intervals.

Each group of animals, control and tested groups, in all experiments consisted of 8-12 mice.

On the fourth or (in case of determination of antibodies type 7S) tenth day after immunisation, mice were slightly anesthetized with ether and exsanguinated by eyeball extirpation. The blood was collected into test tubes. Next, the spinal cord was broken and spleen removed. The blood was used for obtaining the serum needed for determination of hemagglutinating antibodies of the 19S+7S and 7S types, while spleens were used to prepare the cells useful for determination of the percentage of cells able to form E-rosettes and of hemolytic activity. For such uses, mouse spleens were comminuted. The splenocyte cells obtained were suspended in approx. 2 ml of Hanks' medium at +4°C, layered on the Ficoll-Uropolin gradient of a density of 1.077, and then centrifuged for 15 min at 3000 rpm at +4°C. After separation from the interphase, the lymphocyte buffy coat was placed in the Hanks' medium at +4°C and rinsed twice with centrifugation for 7-10 min each time at 1800 rpm. The splenocytes were then suspended in 1 ml of Hanks' medium at such a ratio that it contained 1 x 10⁶ cells.

For each test, the percentage of dead cells is determined by mixing a drop of a tested suspension of splenocytes with a drop of ex tempore prepared dyestuff solution containing 4 parts of a 0.2% trypan blue solution and 1 part of a 4.25% NaCl solution. Under the microscope, the percentage of dead splenocytes is determined for each 100 cells. Dead cells are navy blue, while the bright cells are live cells. The presence of more than 10% of dead cells is critical; such a sample has to be eliminated from further use.

All steps carried out with the cells to be tested should be performed in a sterile, siliconized laboratory glass apparatus placed in an ice bath.

### Example 6:

In the first test, an effect of the tested reaction products on the number of cells producing hemolytic antibodies (PFC-IgM) was determined. The test was carried out as follows: To 0.5 ml of an 0.5% agarose solution placed in a test tube and kept in a heated water bath at 45°C, 0.1 ml of a 10% suspension of SRBC (prepared as described above) were admixed. Then 0.1 ml of a splenocyte suspension having a density of 1 x 10⁶ cells/ml was added, the mixture stirred rapidly and immediately poured out on slides previously covered with agarose. The slides are incubated at 37°C for two hours. Next, the tested samples are covered with guinea pig complement diluted at a ratio of 1:20 for a further 2h. After the incubation of the tested samples with the complement, the number of plaque forming cells (PFC) was counted and recalculated for 1 x 10⁶ splenocytes. Each test was performed twice.

The strongest amplification of the response to SRBC expressed in terms of increase of the number of splenocytes producing hemolysines IgM (PFC) was observed after administration of the D-11 substance at a dose of 0.1 mg/kg. The amplification was 119%. When the daily dose was increased ten times up to 1 mg/kg, amplification of the response was lowered to 53% .

Reaction product D-12 showed the strongest activity - an increase of 58% - at a dose of 1 mg/kg.

Reaction product EK₂-S-11 in this test showed the strongest activity at a dose of 0.1 mg/kg (increase of 65%). At a dose ten times higher, i.e. 1 mg/kg, the increase was slightly lower, i.e. 52%.

Reaction product D-13 tested at a dose of 1 mg/kg caused an increase of 40%. When the dose was increased to 10 mg/kg, i.e. ten times, the response was only a 14% increase.

### Example 7:

An active hemagglutination test was also carried out, determining levels of the anti-SRBC type 19S+7S and 7S antibodies. In order to determine the 19S - IgM type antibodies level, mouse serum was prepared on the fourth day after immunisation with SRBC, while for the determination of 7S-IgG type antibodies level such preparation took place on the tenth day after mice immunisation with SRBC, which is related to the day of maximum count of antibodies of a given type in mice immunised with SRBC.

### A. Determination of 19S+7S antibodies count.

A sample of blood was centrifuged for 30 min at 3500 rpm. From each sample thus prepared, serum was collected and placed in a heated water bath at a temperature of 56°C for 30 min in order to deactivate the complement. Next, a number of solutions at several different dilutions of each tested serum was prepared (from 1:1 to 1:4096) employing a microtitrator and U-shaped microplates having a volume of 250 µl each. The diluted sera were incubated for 1 hour at room temperature. A drop of a 1% suspension of SRBC in PBS (prepared as described above) was added to each serum; the mixtures were incubated for a further 2 h at a temperature of 37°C and then stored at a temperature of +4°C. The results were taken next day. The maximum dilution at which hemagglutination is still caused was considered the antibody count. A ring at the bottom of the plate is a sign that hemagglutination occurs. A button-like formation at the bottom of the plate is considered as a negative result - no hemagglutination.

For statistical analysis of the results, the increase of dilution of serum in a tested substance was compared to the one in a control group.

Reaction product D-11 at a dose of 1 mg/kg increased the IgM count 2.57 times. At a ten times higher dose, the stimulation effect in comparison to the control group increased by 3.5 times.

Reaction product D-12 in this test showed a weaker activity. At a dose of 0.1 mg/kg, it increased the IgM count 2 times, at a dose of 1 mg/kg 1.4 times.

Reaction product EK₂-S-11 showed the strongest activity in this test. At a dose of 0.1 mg/kg, it increased the IgM count 4.3 times, at a dose of 1 mg/kg 3.6 times.

Reaction product D-13 at a dose of 1 mg/kg increased the IgM count 3 times, at a ten times higher dose 1.5 times.

### B. Determination of 7S antibody count

The tested inactivated sera were combined - at a ratio of 1:1 - with a 0.1 M solution of 2-mercaptoethanol and the mixtures incubated for 30 min at a temperature of 37°C. 2-Mercaptoethanol destroys immunoglobulines of the 19S-(IgM) type, while immunoglobulines of the 7S-(IgG) type are not susceptible to the action of 2-mercaptoethanol.

After 30 min of incubation the reduction reaction was stopped by means of cooling down to a temperature of +4°C for 15 min. Next, a number of dilutions was prepared in a similar manner as described above with respect to determining the 19S-antibody count and combined with a 1% suspension of SRBC; after 2 hours of incubation at 37°C, the samples were stored at a temperature of +4°C. The results were evaluated on the following day, according to the criteria of determining the hemagglutination count described above. Simultaneously, a control test was carried out with a combination of a 1% suspension of SRBC with PBS in a ratio of 1:1.

When the substance D-11 was tested as described above, at a dose of 1 mg/kg it increased the production of antibodies IgG 3.16 times. At a dose of 10 mg/kg, the increase was 2.2 times.

Reaction product D-12 tested at a dose of 0.1 mg/kg and 1 mg/kg respectively stimulated the production of antibodies IgG 1.3 times, at a dose of 10 mg/kg 1.5 times.

Reaction product EK₂-S-11 at a dose of 0.1 mg/kg stimulates the production of IgG 1.9 times, at a dose of 1 mg/kg 2.89 times (in comparison with the control).

The results of tests A and B obtained for each production lot or for each fraction of the biologically active reaction products synthesized according to the invention and giving the above-mentioned immunological response were subjected to statistical analysis by the T-student method, α=0.05. Results obtained for each dose tested were compared with a parallel control test and showed an increase of biological activity.

### Example 8:

The group of biologically active reaction products obtained according to Examples 1 to 5 have also been submitted to the test in which the percentage of splenocytes forming E-rosettes was determined.

250 µl of a 1% suspension of SRBC and 250 µl of cells to be tested at a concentration of 1 x 10⁶ cells/ml were added to 550 µl of Hanks' medium. Each such sample was incubated in a heated water bath equipped with a shaker for 15 min at a temperature of 37°C. Then, it was stored at a temperature of +4°C for a further 20 h. The percentage of splenocytes forming E-rosettes with SRBC was determined after the suspension was coloured with 1 to 3 drops of crystal violet.

Each sample was subjected to determination of the percentage of splenocytes three times, counting at each instance 400 splenocytes. A splenocyte surrounded with at least 3 erythrocytes was considered an E-rosette.

For statistical evaluation, the percentage increase of the number of splenocytes with E-rosettes was compared between the substances to be tested and a control group.

In this test, the strongest stimulating effect was shown by the reaction products D-11 (63%) and EK₂-S-11 (70%) at a dose of 1 mg/kg. At a dose ten times smaller, i.e. 0.1 mg/kg, the values decreased to 45% and 57% respectively.

Reaction product D-12 at a dose of 1 mg/kg caused an increase of the ability to form E-rosettes of 22% in comparison to the control group. The respective value for a dose ten times smaller, i.e. of 0.1 mg/kg, was 29%.

Reaction product D-13 shows the maximum effect at a dose of 1 mg/kg (58%), while at higher doses the effect is slightly smaller.

Biological activity of synthesised compounds was evaluated according to the following tests:
1. Test for determination of the percentage of splenocytes forming E-rosettes, carried out according to Bach and Dardenne (Cell. Immunol. 3, 1-16, 1972)
2. Test for determination of the number of cells producing hemolytic antibodies of an IgM type, carried out according to the Jerne method, modified by Mishell and Dutton (J. Exp. Med. 126, 423-442, 1967) and
3. Test for determination of a hemagglutination 19S t 7S and 7S antibody count, carried out according to Adler's active hemagglutination methods (J. Immunol. 95, 26-38, 39-47, 1965) with the use of microplates (J. Immunopharmacol. 4, 43-52, 1982).

### Example 9:

A rotary evaporator flask placed in a heated water bath was charged with:

| | |
|---|---|
| 1.33 g (0.01 M) | L-aspartic acid |
| 0.84 g (0.01 M) | NaHCO₃ |
| 10.00 g | hydrolysed dextrane of an average molecular weight of 3000 daltons |
| 10.00 ml | redistilled water. |

The mixture was heated under pressure at a temperature of 70°C until the solid substances were completely dissolved, expelling by means of distillation during that period approx. 3 ml of water (heating time was approx. 30min). The flask with the solution was loosely covered, placed in a steam steriliser and heated under pressure at a temperature of 121°C for 40 min. After cooling down, the resulting yellow-orange solution was diluted with 15 ml of water, clarified by means of centrifugation and spray dried by air having an inlet temperature of +160°C and an outlet temperature of +85°C. 10.5 g of a light beige reaction product resulted that was easily soluble in water.

The presence of Amadori rearrangement compounds in this reaction product was confirmed by a test by the potassium ferricyanide method described by Borsook, Abrams and Lowy, J. Biol. Soc. 215, (1955), 111-124 and by chromatographic methods.

Biological tests as described in the preceding Examples confirmed an immunotropic activity of the above product similar to the one exhibited by preparations obtained with simple sugars.

### Example 10:

A conical flask was charged with:

| | |
|---|---|
| 5.0 g | hydrolysed dextrane of an average molecular weight of approx. 5000 daltons |
| 1.1 g | L-proline |
| 4.0 ml | redistilled water. |

The content was dissolved by means of stirring. The uniform mixture thus obtained was placed in a steam steriliser and heated under pressure at a temperature of 110°C for 40 min. The resulting transparent orange solution was diluted with 20 ml of redistilled water and clarified by means of centrifugation. The clear solution was spray dried.

5.3 g of a reaction product easily soluble in water was obtained. Immunotropic activity was similar to the one observed in other experiments according to the preceding examples.

### Example 11:

Conventional methods test the biological activity of the compounds in mice, but not in humans. For this reason, new bioassays have been introduced, which measure the amounts and activity of cytokines released from the human peripheral blood leukocytes (PBL) treated with the reaction products according to the Examples 1 to 5, 9 and 10. The cytokines are the hormone-like proteins that play an important role in practically all of the immunological reactions, as well as in the regulatory processes responsible for the maintenance of homeostasis.

Cytotoxicity assays. Cytotoxicity of the reaction products was determined in human lung adenocarcinoma cell line A549 (included in the American Type Culture Collection - ATCC CCL 185). The cell monolayers were trypsinized, suspensions of 2x10⁵ cells/ml in Dulbecco's-modified Eagle's minimum essential medium (DMEM) plus 10% calf serum (CS) were mixed with various doses of the drugs, seeded in the plastic microplates, and incubated for 48 h at 37°C. CD₅₀ was the minimal concentration of the compound which caused approximately 50% destruction of the cell culture, as measured by staining with 0.015% solution of neutral red in DMEM.

Cytokine induction. Buffy coats from healthy blood donors were obtained from the regional transfusion center. The erythrocytes were lysed by NH₄Cl treatment according to Cantell et al. (Cantell, K., Hirvonen, S., Kauppinen, H.L.: Production and Partial Purification of Human Immune Interferon. Meth. Enzymol., 119, 54, 1986). The leukocytes from a single donor containing approximately 8x10⁶ leukocytes/ml in RPMI 1640 medium supplemented with 10% fetal calf serum (FCS), L-glutamine, and antibiotics were used. All lots of FCS were pretested. Only non-mitogenic FCS for PBL cultures was used. The cytokine inducers were added to 1 ml volumes of the cultures. The reference cytokine inducers were phytohemagglutinin (PHA) (Pharmacia Fine Chemicals, Sweden) and LPS from E. coli 0111:B4 (Difco Laboratories). The induced cultures of PBL were incubated in an atmosphere of 5% CO₂ in air at 37°C for 20 h and centrifuged. Supernatants were stored at 4°C and assayed for TNF and IFN activity within one week.

Interferon (IFN) assay. The confluent monolayer of A549 cells was prepared in the microplates in DMEM with 10% CS, L-glutamine, and antibiotics (100 units/ml penicillin and 100 µg/ml streptomycin). IFN samples diluted in plates were added to the cell monolayer and incubated at 37°C for 20 h in 5% CO₂ in air. The cells were then washed and challenged with encephalomyocarditis virus (EMCV). The titer of IFN was defined as the dilution of IFN sample that reduced the virus cytopathogenic effect by 50% after 48 h of incubation. The MTT (3-[4,5-dimethylthiazol-2-yl]-2,5- diphenyltetrazolium bromide) method (Hansen, M.B., Nielsen, S.E., and Berg, K.: Re-examination and Further Development of a Precise and Rapid Dye Method fcr Measuring Cell Growth/ Cell Kill. J. Immuno. Meth., 1989, 119, 203-210) to measure the cell kill in the ELISA scanner was also used. Laboratory standards of IFNs were included in all assays: Recombinant human IFN-γ (Genentech Inc., USA, specific activity 2x10⁸ units/mg), the natural human leukocyte IFN-α (3x10⁶ IU/ml) and IFN-γ (2x10⁶ IU/ml) obtained from Dr. K. Cantell, Helsinki, Finland.

Tumor Necrosis Factor (TNF) assay. The cytotoxic activity of TNF was measured in L929 cells according to Flick and Gifford (Flick, D.A., Gifford, G.E.: Comparison of in Vitro Cell Cytotoxic Assays for Tumor Necrosis Factor. J. Immunol. Meth., 68, 167, 1984).

The samples and actinomycin D solution were added to monolayer cultures of the cells. After incubation at 37°C for 20 h, the cultures were stained with crystal violet and toxic effects were determined. The amount causing approximately 50% destruction of the cell cultures was defined as one unit of TNF activity. Comparison with a preparation of TNF-a (Genentech Inc., USA) showed that 1 unit in our assays was equal to 100 - 200 pg/ml TNF.

Cytokine neutralization assays. The antisera used were: rabbit anti-human TNF-α, lot 2958-40 and rabbit anti-human IFN-γ, lot 2891-56 (Genentech Inc., USA), sheep anti-human IFN-α,β, and sheep anti-human IFN-γ (obtained from Dr. K. Cantell, Finland). The cytokine preparations were treated with the sera diluted 1:200 in culture medium and incubated for one hour at room temperature. Then, the residual IFN or TNF activities were determined as described.

Five different batches (L₁ to L₅) of PBL prepared from the blood of healthy blood donors were used. The optimal PBL concentration for the assays was found to be 8x10⁶ cells per one ml of medium (RPMI 1640 supplemented with 10% fetal calf serum and antibiotics).

Incubation of human PBL with the new reaction products I - XI (Table 1) resulted in IFN and TNF synthesis. The observed responses were dose related in the range of 3 - 100 µg/ml of the compounds (Table 2). The compounds used in the indicated concentrations were non-cytotoxic. In all of the bioassays, the negative and positive controls were included. The negative controls measured the amounts of the cytokines (IFN and TNF) produced spontaneously without the addition of any exogenous stimulants. The positive controls indicated the amounts of the cytokine produced in response to a known reference inducer; in our case this was phytohemagglutinin (PHA, Pharmacia, Sweden, 10µg per ml).

It should be pointed out that the cytokine induction in human PBL cultures obtained from different individuals usually gives considerable variation of the results. The phenomenon may be explained in terms of genetic differentiation of human population. Furthermore, PBL cultures often produce IFN and TNF spontaneously.

In other words, high responders and low responders or even non-responders are commonly observed among the healthy donors of PBL.

Despite the presented limitations, the results of the bioassays showed that PBL (L₁ to L₅) treated with the reaction products (I - XI) produced IFN and/or TNF that could be measured quantitatively.

In the case of L₁ which contained leukocytes of the high responder, the reaction product II (containing L-form of aspartic acid) was found to be considerably more active as a cytokine inducer than the reaction product III (containing the D-form of aspartic acid which also is more expensive by two orders of magnitude). The observation is significant because mainly L-forms of the amino acids are recognized by cells as natural substrates in biochemical reactions.

Furthermore, for the expression of biological activity of the reaction products, the amino acid part of the molecule is much more important than the form of sugar. Instead of single sugars, - preferably low molecular weight, especially of less than 1000 daltons - polysaccharides (such as dextranes, reaction products X - XI) can be used and they react similarly.

And vice versa, polysaccharides containing the amino acid residues may have biological activity, and this activity is retained when they are decomposed to oligosaccharides with the bound amino acid (data not shown).

Similar results may also be observed if a short peptide is taken instead of a single amino acid and is used to stimulate the leukocytes to produce cytokines (data not shown).

Seven reference batches of the unfractionated TTP assayed in over 100 PBL cultures from different donors produced from 10 to 1.000 units of IFN per ml, and from 9 to 750 units of TNF per ml. The fraction EK₂-S prepared from a mixture corresponding to the contents of natural peat extract (Example 5) has been assayed in eight PBL cultures from eight different blood donors. It was found to be the most active preparation in inducing both IFN and TNF (data not shown).

Possible applications of the reaction products are as immunomodulators and such activity was clinically useful. Tissue regeneration is another proven activity. Anti-cancer activity probably connected with the presence of the induced interferon and tumor necrosis factor was also observed. Anti-viral activity was also noted.

The main use of the above reaction products involves oral administration, but parenteral treatment is also possible, as is topical application. The products appear relatively stable.

**Table 1.**

| List of Reaction Products | |
|---|---|
| No. | Substrates |
| I (D-10) | L-glutamic acid, glucose, galactose |
| II (D-11) | L-aspartic acid, glucose, galactose |
| III (D-13) | D-aspartic acid, glucose, galactose |
| IV (D-12) | L-serine, glucose, galactose |
| V | EK₂-S (fractions 11 - 13) |
| VI | EK₂-S (fractions 6 - 7) |
| VII | EK₂-S (fractions 8 - 10) |
| VIII | EK₂-S (fractions 28 - 34) |
| IX | L-proline, glucose |
| X | L-aspartic acid + dextrane (variety 1) |
| XI | L-aspartic acid + dextrane (variety 2) |

### Example 12:

Pharmaceutical formulations containing as an active ingredient the reaction products according to Examples 1 to 5, 9 and 10, were prepared using the following reaction products:

| A. Tablets/Granules: | |
|---|---|
| 5.0 g | of the reaction product obtained according to Example 1 or 10 (active substance), |
| 444.0 g | of pharmaceutically acceptabie lactose |
| 1.0 g | of lubricant, e.g. MYVATEX^{(R)}, trademark of Eastman Kodak) |

The ingredients were mixed and granulated with 30% aqueous ethanol in a conventional way, then dried at 40°C. The granules were used to prepare capsules, each containing approx. 450 mg of granules, i.e. 5 mg of the active substance. Alternatively, the granules were used to form tablets, each weighing approx. 450 mg and containing 5 mg of the active substance.
B. In the same conventional manner, the active substances obtained according to the preceding examples 1 to 5, 9 and 10 were formulated into other pharmaceutical formulations using suitable carriers.

**Table 2.**

| Biological Activity of the Reaction Products I-XI in Human PBL | | | | | | | | | | | |
|---|---|---|---|---|---|---|---|---|---|---|---|
| | Dose µg/ml | IFN units/ml | | | | | TNF units/ml | | | | |
| | | L₁ | L₂ | L₃ | L₄ | L₅ | L₁ | L₂ | L₃ | L₄ | L₅ |
| Control | - | 10 | <10 | <10 | 20 | <10 | 80 | 9 | 27 | 27 | <9 |
| PHA | 10 | 100 | 30 | 30 | 60 | 100 | 250 | 80 | 250 | 250 | 250 |
| I | 100 | 100 | <10 | <10 | 20 | - | 250 | 9 | 9 | 160 | - |
| | 30 | - | - | - | 30 | - | - | - | - | 500 | - |
| | 10 | 30 | <10 | <10 | 30 | - | 80 | 9 | 18 | 160 | - |
| | 3 | - | - | - | 10 | - | - | - | - | 160 | - |
| II | 100 | 100 | 10 | <10 | 10 | <10 | 250 | 18 | 18 | 250 | <9 |
| | 30 | - | - | - | 10 | <10 | - | - | - | 250 | <9 |
| | 10 | 1000 | 10 | 30 | 10 | 10 | 250 | 50 | 27 | 250 | <9 |
| | 3 | - | - | - | 10 | <10 | - | - | - | 160 | <9 |
| III | 100 | <10 | <10 | 10 | 10 | <10 | 250 | 9 | 18 | 250 | <9 |
| | 30 | - | - | - | 30 | <10 | - | - | - | 250 | <9 |
| | 10 | <10 | <10 | <10 | 10 | <10 | 80 | 27 | 18 | 250 | <9 |
| | 3 | - | - | - | 10 | <10 | - | - | - | 80 | <9 |
| IV | 100 | <10 | 10 | 10 | 20 | <10 | 80 | 60 | 9 | 160 | <9 |
| | 30 | - | - | - | 20 | <10 | - | - | - | 27 | <9 |
| | 10 | <10 | 30 | <10 | 20 | <10 | 80 | 50 | 18 | 80 | <9 |
| | 3 | - | - | - | 20 | <10 | - | - | - | 80 | <9 |
| V | 100 | 30 | <10 | <10 | 60 | - | 80 | 50 | 18 | 250 | - |
| | 30 | - | - | - | 60 | - | - | - | - | 80 | - |
| | 10 | <10 | <10 | <10 | 10 | - | 80 | 27 | 9 | 80 | - |
| | 3 | - | - | - | 10 | - | - | - | - | 80 | - |
| VI | 100 | <10 | <10 | <10 | 20 | - | 80 | 27 | 18 | 80 | - |
| | 30 | - | - | - | 10 | - | - | - | - | 80 | - |
| | 10 | 10 | <10 | <10 | 20 | - | 50 | 27 | 18 | 160 | - |
| | 3 | - | - | - | 10 | - | - | - | - | 250 | - |
| VII | 100 | <10 | <10 | <10 | - | - | 80 | 27 | 27 | - | - |
| | 30 | - | - | - | - | - | - | - | - | - | - |
| | 10 | 10 | <10 | <10 | - | - | 80 | 27 | 27 | - | - |
| | 3 | - | - | - | - | - | - | - | - | - | - |
| VIII | 100 | <10 | <10 | <10 | - | - | 80 | 160 | 27 | - | - |
| | 30 | - | - | - | - | - | - | - | - | - | - |
| | 10 | <10 | <10 | <10 | - | - | 750 | 80 | 27 | - | - |
| | 3 | - | - | - | - | - | - | - | - | - | - |
| IX | 100 | 10 | <10 | <10 | 10 | - | 27 | 27 | 18 | 80 | - |
| | 30 | - | - | - | 20 | - | - | - | - | 80 | - |
| | 10 | 100 | 30 | <10 | 20 | - | 80 | 27 | 18 | 80 | - |
| | 3 | - | - | - | 10 | - | - | - | - | 50 | - |
| X | 100 | 100 | <10 | 20 | 20 | - | 27 | 27 | 18 | 250 | - |
| | 30 | - | - | - | 30 | - | - | - | - | 80 | - |
| | 10 | <10 | 10 | <10 | 30 | - | 18 | 50 | 27 | 50 | - |
| | 3 | - | - | - | 30 | - | - | - | - | 250 | - |
| XI | 100 | <10 | 10 | <10 | 30 | - | 18 | 27 | 27 | 50 | - |
| | 30 | - | - | - | 10 | - | - | - | - | 250 | - |
| | 10 | <10 | 30 | <10 | 10 | - | 18 | 80 | 80 | 80 | - |
| | 3 | - | - | - | 20 | - | - | - | - | 750 | - |

### Example 13:

The active substances obtained according to preceding Examples 1 to 5, 9 and 10 were used as a beneficial additive to cosmetic preparations intended for everyday hair and body care, the content of the substances being within a range of 0.01 - 10% by weight, depending on the type of the preparation, the method of application and the frequency of use intended for the particular preparation.

## Claims

1. Use of at least one Amadori rearrangement compound, preferably a mixture of Amadori rearrangement compounds, of the general formula
R₁-NH-R₂,
wherein R₁ comprises a 1-deoxy-2-ketose radical derived from a simple sugar, oligo- or polysaccharide and R₂ comprises an amino acid or peptide radical, for the manufacture of a pharmaceutical composition to treat or prevent hematological diseases and/or to induce cytokine formation.

2. Use according to claim 1 characterized in that the composition comprises a mixture of at least two Amadori rearrangement compounds having different 1-deoxy-2-ketose radicals and/or different amino acid or peptide radicals.

3. Use according to claim 1 or 2, wherein the sugar radical is in its D-form, preferably selected from the group consisting of glucose, xylose, galactose, rhamnose, fructose, mannose, 2-deoxy-glucose, 6-deoxy-glucose, glucosamine, and galactosamine.

4. Use according to anyone of claims 1 to 3, wherein the amino acid radical is in its L-form, preferably selected from the group consisting of serine, glycine, proline, histidine, arginine, alanine, aspartic acid, glutamic acid, phenylalanine, threonine, cysteine, cystine, glutamine, valine, asparagine, methionine, tyrosine, hydroxyproline, lysine, tryptophane, isoleucine, and leucine.

5. Use according to any one of claims 1 to 4, wherein the oligo- or polysaccharide radical has a molecular weight of 5000 dalton or less, especially of less than 1000 dalton and/or the peptide radical has a molecular weight of less than 1000 dalton.

6. Use according to any one of claims 1 to 5, wherein the radicals are present in essentially the same composition and/or in essentially the same weight ratio as occurring in natural peat extract, and wherein said composition optionally further comprises inorganic trace elements occurring in said extract.

7. Use according to any one of claims 1 to 6, wherein the composition further comprises at least one pharmaceutically acceptable additive selected from the group consisting of a carrier, an adjuvant and a lubricant, in a weight ratio of said Amadori rearrangement compounds to said additive of between 1:1 to 1:100, preferably 1:8 to 1:20, and most preferably about 1:9.

8. Use according to any one of claims 1 to 6, wherein the composition further comprises at least one acceptable additive selected from the group consisting of a carrier, an adjuvant and a lubricant, and wherein the Amadori rearrangement compounds are present in an amount of 0.01 - 10% by weight.

9. Use according to claim 7 or 8, wherein said lubricant is present in admixture with lactose, the weight ratio of lactose to the lubricant being between 20:1 and 100:1, preferably about 50:1.

10. Use according to claim 8 or 9, wherein the Amadori rearrangement compounds are present in an amount of 0.01 - 1%, preferably 0.05-0.1 % by weight.

11. Process for the manufacture of at least one Amadori rearrangement compound, preferably a mixture of Amadori rearrangement compounds, of the general formula
R₁-NH-R₂,
wherein R₁ comprises a 1-deoxy-2-ketose radical derived from a simple sugar, oligo- or polysaccharide and R₂ comprises an amino acid or peptide radical, characterized in that said process comprises
a) reacting at least one amino acid or peptide, preferably at least two different amino acids or peptides, with at least one simple sugar, oligo- or polysaccharide, preferably with at least two different sugars, oligo- or polysaccharides, wherein the molar ratio of sugars and/or saccharides to the amino acids and/or peptides is between 2:1 and 1:1, and preferably is 1.5:1, in a concentrated aqueous solution of about 0.67 to about 2.75 pbw of solids per 1 pbw of aqueous solvent at a temperature of about 70-121°C,
b) continuing heating to allow the resulting products to undergo Amadori rearrangement while simultaneously or subsequently removing the aqueous solvent, until - preferably after about 30 to 120 minutes - the reaction mixture turns light orange-brown in colour and biological activity and ferricyanide reducing capacity can be detected in samples taken from the mixture,
c) stopping the Amadori rearrangement, preferably before decomposition occurs yielding compounds that have lost their biological activity, and
d) drying the reaction mixture and/or further subjecting it to a purification by column chromatography wherein biologically active fractions causing reduction of potassium ferricyanide are collected.

12. Process according to claim 11, wherein the reaction is carried out in the presence of inorganic trace elements occurring in natural peat extract and optionally under pressure and/or in the presence of a lower alcohol.

13. Process according to claim 11 or 12, wherein the oligo- or polysaccharides have a molecular weight of 5000 daltons or less and/or the peptides have a molecular weight of less than 1000 daltons.

14. Process according to anyone of claims 11 to 13 wherein the simple sugars, oligo- or polysaccharides, amino acids and/or peptides, and optionally the inorganic trace elements, are present in essentially the same composition and/or in essentially the same weight ratio as occurring in natural peat extract.

15. Process according to anyone of claims 11 to 14, wherein at least one amino acid has two carboxyl groups and the reaction is carried out in the presence of a buffer salt, preferably sodium bicarbonate in a molar ratio with the amino acid of 1:1.

16. A mixture of Amadori rearrangement compounds of the general formula
R₁-NH-R₂,
wherein R₁ comprises a 1-deoxy-2-ketose radical derived from a simple sugar, oligo- or polysaccharide and R₂ comprises an amino acid or peptide radical, characterized in that the radicals are present in essentially the same composition and/or in essentially the same weight ratio as occurring in natural peat extract.

17. A mixture according to claim 16, characterized in that it further comprises inorganic trace elements occurring in natural peat extract.

18. A mixture according to claim 16 or 17, obtainable in a process as defined in claim 14.

19. A mixture according to anyone of claims 16 to 18, for medical or cosmetic use, preferably for use as an immunomodulating, particularly cytokine inducing, agent.

20. A pharmaceutical or cosmetic composition comprising a mixture of Amadori rearrangement compounds as defined in anyone of claims 16 to 19, in combination with at least one pharmaceutically or cosmetically acceptable additive selected from the group consisting of a carrier, an adjuvant and a lubricant.

21. A pharmaceutical composition according to claim 20, characterized in that the weight ratio of said Amadori rearrangement compounds to said pharmaceutically acceptable additive ranges from 1:1 to 1:100, preferably from 1:8 to 1:20, and most preferably is about 1:9.

22. A cosmetic composition according to claim 20, characterized in that the Amadori rearrangement compounds are present in an amount of 0.01 - 10%, preferably 0.01 - 1%, most preferably 0.05-0.1 % by weight.

23. Use according to any one of claims 1 to 10, wherein said Amadori rearrangement compound or said mixture of Amadori rearrangement compounds is part of a biologically active reaction mixture obtainable by a process as defined in any one of claims 11 to 15.

## Patentansprüche

1. Verwendung wenigstens einer Amadori-Umlagerungsverbindung, vorzugsweise eines Gemisches von Amadori-Umlagerungsverbindungen, der allgemeinen Formel
R₁-NH-R₂
worin R₁ eine von einem einfachen Zucker, einem Oligo- oder einem Polysaccharid abgeleitete 1-Deoxy-2-Ketose-Radikale umfasst und R₂ eine Aminosäuren- oder Peptid-Radikale aufweist, für die Herstellung einer pharmazeutischen Zusammensetzung zum Behandeln oder Verhindern hämatologischer Erkrankungen und/ oder zum Induzieren einer Zytokin-Bildung.

2. Verwendung nach Anspruch 1, dadurch gekennzeichnet, dass die Zusammensetzung ein Gemisch von wenigstens zwei Amadori-Umlagerungsverbindungen mit unterschiedlichen 1-Deoxy-2-Ketose-Radikalen und/oder unterschiedlichen Aminosäuren- oder Peptid-Radikalen aufweist.

3. Verwendung nach Anspruch 1 oder 2, bei der die Zuckerradikale in ihrer D-Form vorliegt und vorzugsweise aus der aus Glukose, Xylose, Galaktose, Rhamnose, Fruktose, Mannose, 2-Deoxy-Glukose, 6-Deoxy-Glukose, Glukosamin und Galaktosamin bestehenden Gruppe ausgewählt ist.

4. Verwendung nach einem der Ansprüche 1 bis 3, bei der die Aminosäuren-Radikale in ihrer L-Form vorliegt und vorzugsweise aus der aus Serin, Glycin, Prolin, Histidin, Arginin, Alanin, Asparaginsäure, Glutaminsäure, Phenylalanin, Threonin, Cystein, Cystin, Glutamin, Valin, Asparagin, Methionin, Tyrosin, Hydroxyprolin, Lysin, Tryptophan, Isoleucin und Leucin bestehenden Gruppe ausgewählt ist.

5. Verwendung nach einem der Ansprüche 1 bis 4, bei der die Oligo- oder Polysaccharid-Radikale ein Molekulargewicht von 5000 Dalton oder weniger hat, insbesondere weniger als 1000 Dalton, und/oder die Peptid-Radikale ein Molekulargewicht von weniger als 1000 Dalton besitzt.

6. Verwendung nach einem der Ansprüche 1 bis 5, bei der die Radikalen in im wesentlichen derselben Zusammensetzung und/ oder in im wesentlichen demselben Gewichtsverhältnis vorliegen, wie sie in natürlichem Torf-Extrakt vorkommen, und bei der die Zusammensetzung gegebenenfalls des weiteren anorganische Spurenelemente aufweist, die in diesem Extrakt vorkommen.

7. Verwendung nach einem der Ansprüche 1 bis 6, bei der die Zusammensetzung ferner zumindest ein pharmazeutisch akzeptables, aus der aus einem Träger, einem Hilfsmittel und einem Schmiermittel bestehenden Gruppe ausgewähltes Additiv in einem Gewichtsverhältnis der Amadori-Umlagerungsverbindungen zu dem Additiv zwischen 1:1 bis 1:100, vorzugsweise 1:8 bis 1:20, und am bevorzugtesten von etwa 1:9, aufweist.

8. Verwendung nach einem der Ansprüche 1 bis 6, bei der die Zusammensetzung ferner zumindest ein akzeptables, aus der aus einem Träger, einem Hilfsmittel und einem Schmiermittel bestehenden Gruppe ausgewähltes Additiv aufweist und die Amadori-Umlagerungsverbindungen in einer Menge von 0,01 - 10 Gew.-% vorliegen.

9. Verwendung nach Anspruch 7 oder 8, bei der das Schmiermittel in einer Mischung mit Laktose vorliegt, wobei das Gewichtsverhältnis von Laktose zum Schmiermittel zwischen 20:1 und 100:1, vorzugsweise von etwa 50:1, beträgt.

10. Verwendung nach Anspruch 8 oder 9, bei der die Amadori-Umlagerungsverbindungen in einer Menge von 0,01 - 1%, vorzugsweise von 0,05-0,1 Gew.-%, vorliegen.

11. Verfahren zur Herstellung wenigstens einer Amadori-Umlagerungsverbindung, vorzugsweise eines Gemisches von Amadori-Umlagerungsverbindungen, der allgemeinen Formel
R₁-NH-R₂
worin R₁ eine von einem einfachen Zucker, einem Oligo- oder einem Polysaccharid abgeleitete l-Deoxy-2-Ketose-Radikale umfasst und R₂ eine Aminosäuren- oder Peptid-Radikale aufweist, dadurch gekennzeichnet, dass das Verfahren folgendes umfasst:
a) Umsetzen wenigstens einer Aminosäure oder wenigstens eines Peptides, vorzugsweise von wenigstens zwei unterschiedlichen Aminosäuren oder Peptiden, mit mindestens einem einfachen Zucker, Oligo- oder Polysaccharid, vorzugsweise mit mindestens zwei unterschiedlichen Zuckern, Oligo- oder Polysacchariden, wobei das molare Verhältnis der Zucker und/ oder Sacchariden zu den Aminosäuren und/oder Peptiden zwischen 2:1 und 1:1 liegt, und vorzugsweise 1,5:1 beträgt, in einer konzentrierten wässrigen Lösung von etwa 0,67 zu ungefähr 2,75 pbw Feststoffen pro 1 pbw wässrigen Lösungsmittels bei einer Temperatur von etwa 70-121°C,
b) Fortsetzen des Erhitzens, um es den sich ergebenden Produkten zu gestatten, eine Amadori-Umlagerung durchzumachen, während das wässrige Lösungsmittel gleichzeitig oder darauf folgend entfernt wird, bis - vorzugsweise nach etwa 30 bis 120 Minuten - sich die Reaktionsmischung orange-braun verfärbt und die biologische Aktivität und die Ferricyanid reduzierende Fähigkeit in dem Gemisch entnommenen Proben festgestellt werden kann,
c) Beenden der Amadori-Umlagerung, vorzugsweise vor einer Zersetzung, welche Verbindungen, die ihre biologische Aktivität verloren haben, vergilbt, und
d) Trocknen der Reaktionsmischung und/oder Unterziehen derselben des weiteren einer Reinigung durch Kolonnen-Chromatographie, wobei biologisch aktive, eine Reduktion von Kaliumferricyanid verursachende Fraktionen gesammelt werden.

12. Verfahren nach Anspruch 11, bei welchem die Reaktion in Gegenwart anorganischer Spurenelemente durchgeführt wird, welche in natürlichem Torfextrakt vorkommen, und gegebenenfalls unter Druck und/oder in Gegenwart eines niedrigen Alkohols.

13. Verfahren nach Anspruch 11 oder 12, bei welchem die Oligo- oder Polysaccharide ein Molekulargewicht von 5000 Dalton oder weniger haben und/oder die Peptide ein Molekulargewicht von weniger als 1000 Dalton besitzen.

14. Verfahren nach einem der Ansprüche 11 bis 13, bei welchem die einfachen Zucker, Oligo- oder Polysaccharide, Aminosäuren und/oder Peptide, und gegebenenfalls die anorganischen Spurenelemente, in im wesentlichen derselben Zusammensetzung und/ oder in im wesentlichen demselben Gewichtsverhältnis vorliegen, wie sie in natürlichem Torf-Extrakt vorkommen.

15. Verfahren nach einem der Ansprüche 11 bis 14, bei welchem wenigstens eine Aminosäure zwei Carboxylgruppen hat und die Reaktion in Gegenwart eines Puffersalzes, vorzugsweise von Natriumbicarbonat, in einem molaren Verhältnis zur Aminosäure von 1:1 durchgeführt wird.

16. Gemisch von Amadori-Umlagerungsverbindungen, der allgemeinen Formel
R₁-NH-R₂
worin R₁ eine von einem einfachen Zucker, einem Oligo- oder einem Polysaccharid abgeleitete l-Deoxy-2-Ketose-Radikale umfasst und R₂ eine Aminosäuren- oder Peptid-Radikale aufweist, dadurch gekennzeichnet, dass die Radikalen in im wesentlichen derselben Zusammensetzung und/oder in im wesentlichen demselben Gewichtsverhältnis vorliegen, wie sie in natürlichem Torf-Extrakt vorkommen.

17. Gemisch nach Anspruch 16, dadurch gekennzeichnet, dass es des weiteren anorganische Spurenelemente aufweist, die in natürlichem Torfextrakt vorkommen.

18. Gemisch nach Anspruch 16 oder 17, welches durch ein Verfahren nach Anspruch 14 erhältlich ist.

19. Gemisch nach einem der Ansprüche 16 bis 18 für den medizinischen oder kosmetischen Gebrauch, vorzugsweise für die Verwendung als immunmodulierendes, insbesondere Zytokin induzierendes, Mittel.

20. Pharmazeutische oder kosmetische Zusammensetzung mit einem Gemisch von Amadori-Umlagerungsverbindungen nach einem der Ansprüche 16 bis 19 in Kombination mit zumindest einem pharmazeutisch oder kosmetisch akzeptablen, aus der aus einem Träger, einem Hilfsmittel und einem Schmiermittel bestehenden Gruppe ausgewählten Additiv.

21. Pharmazeutische Zusammensetzung nach Anspruch 20, dadurch gekennzeichnet, dass das Gewichtsverhältnis der Amadori-Umlagerungsverbindungen zu dem pharmazeutisch akzeptablen Additiv von 1:1 bis 1:100, vorzugsweise von 1:8 bis 1:20, reicht, und am bevorzugtesten etwa 1:9 beträgt.

22. Kosmetische Zusammensetzung nach Anspruch 20, dadurch gekennzeichnet, dass die Amadori-Umlagerungsverbindungen in einer Menge von 0,01 - 10%, vorzugsweise von 0,01 - 1%, und am bevorzugtesten von 0,05-0,1 Gew.-%, vorliegen.

23. Verwendung nach einem der Ansprüche 1 bis 10, bei der die Amadori-Umlagerungsverbindung oder das Gemisch von Amadori-Umlagerungsverbindungen Teil einer biologisch aktiven Reaktionsmischung ist, welche durch ein Verfahren nach einem der Ansprüche 11 bis 15 erhältlich ist.

## Revendications

1. Utilisation d'au moins un composé issu d'un réarrangement d'Amadori, de préférence d'un mélange de composés issus d'un réarrangement d'Amadori, de la formule générale :
R₁-NH-R₂
dans laquelle R₁ comprend un radical 1-désoxy-2-cétose dérivé d'un sucre simple, d'un oligo ou d'un polysaccharide et R₂ comprend un radical acide aminé ou peptide, pour la préparation d'une composition pharmaceutique pour traiter ou prévenir des maladies hématologiques et/ou pour induire la formation de cytokine.

2. Utilisation selon la revendication 1, caractérisée en ce que la composition comprend un mélange d'au moins deux composés issus d'un réarrangement d'Amadori ayant des radicaux 1-désoxy-2-cétose différents et/ou des radicaux acide aminé ou peptide différents.

3. Utilisation selon la revendication 1 ou 2, dans laquelle le radical sucre est sous la forme D, en étant choisi, de préférence, dans le groupe constitué par le glucose, le xylose, le galactose, le rhamnose, le fructose, le mannose, le 2-désoxy-glucose, le 6-désoxy-glucose, la glucosamine et la galactosamine.

4. Utilisation selon l'une quelconque des revendications 1 à 3, dans laquelle le radical acide aminé est sous sa forme L et est choisi de préférences dans le groupe constitué par la sérine, la glycine, la proline, l'histidine, l'arginine, l'alanine, l'acide aspartique, l'acide glutamique, la phénylalanine, la thréonine, la cystéine, la cystine, la glutamine, la valine, l'asparagine, la méthionine, la tyrosine, l'hydroxyproline, la lysine, le tryptophane, l'isoleucine et la leucine.

5. Utilisation selon l'une quelconque des revendications 1 à 4, dans laquelle le radical oligo- ou polysaccharide a un poids moléculaire de 5000 daltons ou moins, en particulier inférieur à 1000 daltons et/ou le radical peptide a un poids moléculaire de moins de 1000 daltons.

6. Utilisation selon l'une quelconque des revendications 1 à 5, dans laquelle les radicaux sont présents en ayant sensiblement la même composition que celle dans un extrait de tourbe naturelle et/ou sont présents sensiblement dans le même rapport pondéral que dans cet extrait, et dans laquelle ladite composition comprend, en outre et à titre facultatif, des éléments inorganiques présents à l'état de traces dans cet extrait.

7. Utilisation selon l'une quelconque des revendications 1 à 6, dans laquelle la composition comprend, en outre, au moins un additif acceptable sur le plan pharmaceutique, choisi dans le groupe constitué par un vecteur, un adjuvant et un lubrifiant, avec un rapport pondéral desdits composés issus d'un réarrangement d'Amadori sur ledit additif entre 1 : 1 et 1 : 100, de préférence entre 1 : 8 et 1 : 20 et surtout d'environ 1 : 9.

8. Utilisation selon l'une quelconque des revendications 1 à 6, dans laquelle la composition comprend, en outre, au moins un additif acceptable sur le plan pharmaceutique, choisi dans le groupe constitué par un vecteur, un adjuvant et un lubrifiant et dans laquelle les composés issus d'un réarrangement d'Amadori sont présents en une quantité de 0,01 à 10 % en poids.

9. Utilisation selon la revendication 7 ou 8, dans laquelle ledit lubrifiant est présent en mélange avec du lactose, le rapport pondéral du lactose sur le lubrifiant étant entre 20 : 1 et 100 : 1 et, de préférence, d'environ 50 : 1.

10. Utilisation selon la revendication 8 ou 9, dans laquelle les composés issus d'un réarrangement d'Amadori sont présents en une quantité de 0,01 - 1 % et, de préférence, de 0,05 - 0,1 % en poids.

11. Procédé de préparation d'au moins un composé issu d'un réarrangement d'Amadori et, de préférence, d'un mélange de composés issus d'un réarrangement d'Amadori de la formule générale
R₁-NH-R₂,
dans laquelle R₁ comprend un radical 1-désoxy-2-cétose dérivé d'un sucre simple, d'un oligo- ou d'un polysaccharide et R₂ comprend un radical acide aminé ou peptide, caractérisé en ce que ledit procédé comprend les étapes consistant à :
a) faire réagir au moins un acide aminé ou un peptide et, de préférence, au moins deux acides aminés ou peptides différents avec au moins un sucre simple, un oligo- ou un polysaccharide et, de préférence, avec au moins deux sucres, oligo- ou polysaccharides différents, le rapport molaire des sucres et/ou des saccharides sur les acides aminés et/ou les peptides étant entre 2 : 1 et 1 : 1 et, de préférence, de 1,5 : 1, dans une solution aqueuse concentrée constituée par environ 0,67 à environ 2,75 parties en poids de matières solides par partie en poids de solvant aqueux, à une température d'environ 70 - 121 °C,
b) continuer de chauffer, de préférence pendant environ 30 à 120 minutes, pour permettre aux produits obtenus de subir un réarrangement d'Amadori, tout en évacuant en même temps ou subséquemment le solvant aqueux, jusqu'à ce que le mélange réactionnel prenne une teinte orange-brun légère et qu'une activité biologique et une capacité à réduire un ferricyanure puissent être détectées dans des échantillons pris dans le mélange,
c) arrêter le processus de réarrangement d'Amadori, de préférence avant qu'une décomposition ne se produise, aboutissant à des composés qui ont perdu leur activité biologique, et à
d) sécher le mélange réactionnel et/ou le soumettre à une purification sur une colonne de chromatographie, permettant de recueillir des fractions biologiquement actives et capables de réduire le ferricyanure de potassium.

12. Procédé selon la revendication 11, dans lequel la réaction est effectuée en présence d'éléments inorganiques présents à l'état de traces dans l'extrait de tourbe naturelle et, éventuellement, sous pression et/ou en présence d'un alcool inférieur.

13. Procédé selon la revendication 11 ou 12, dans lequel les oligo- ou les polysaccharides ont un poids moléculaire de 5000 daltons ou moins et/ou les peptides ont un poids moléculaire inférieur à 1000 daltons.

14. Procédé selon l'une quelconque des revendications 11 à 13, dans lequel les sucres simples, les oligo- ou les polysaccharides, les acides aminés et/ou les peptides et, éventuellement, des éléments inorganiques à l'état de traces sont présents en ayant sensiblement la même composition que dans les extraits de tourbe naturelle et/ou sensiblement dans le même rapport pondéral que dans cet extrait.

15. Procédé selon l'une quelconque des revendications 11 à 14, dans lequel au moins un des acides aminés a deux groupes carboxyle et dans lequel la réaction s'effectue en présence d'un sel tampon, de préférence le bicarbonate de sodium, dans un rapport molaire sur l'acide aminé de 1 : 1.

16. Mélange de composés issus d'un réarrangement d'Amadori de la formule générale
R₁-NH-R₂
dans laquelle R₁ comprend un radical 1-désoxy-2-cétone dérivé d'un sucre simple, d'un oligo- ou d'un polysaccharide et R₂ comprend un radical acide aminé ou peptide, caractérisé en ce que la composition des radicaux présents est sensiblement la même que dans l'extrait de tourbe naturelle et/ou que leur rapport pondéral est sensiblement le même que dans cet extrait.

17. Mélange selon la revendication 16, caractérisé en ce qu'il comprend en outre, les éléments inorganiques se trouvant à l'état de traces dans un extrait de tourbe naturelle.

18. Mélange selon la revendication 16 ou 17, qui peut être obtenu dans le procédé comme défini dans la revendication 14.

19. Mélange selon l'une quelconque des revendications 16 à 18, pour un usage médical ou cosmétique, de préférence comme agent immunomodulateur, en particulier comme agent inducteur de la cytokine.

20. Composition pharmaceutique ou cosmétique comprenant des composés issus d'un réarrangement d'Amadori comme défini dans l'une quelconque des revendications 16 à 19, en combinaison avec au moins un additif acceptable sur le plan pharmaceutique ou sur le plan cosmétique, et choisi dans le groupe constitué par un vecteur, un adjuvant et un lubrifiant.

21. Composition pharmaceutique selon la revendication 20, caractérisée en ce que le rapport pondéral desdits composés issus d'un réarrangement d'Amadori sur ledit additif acceptable sur le plan pharmaceutique se situe dans la plage de 1 : 1 à 1 : 100, de préférence de 1 : 8 à 1 : 20 et surtout, il est d'environ 1 : 9.

22. Composition cosmétique selon la revendication 20, caractérisée en ce que les composés issus du réarrangement d'Amadori sont présents à raison de 0,01 - 10 %, de préférence de 0,01 - 1 % et surtout de 0,05 - 0,1 % en poids.

23. Utilisation selon l'une quelconque des revendications 1 à 10, dans laquelle ledit composé issu d'un réarrangement d'Amadori ou ledit mélange de composés issus d'un réarrangement d'Amadori fait partie d'un mélange réactionnel biologiquement actif qui peut être obtenu par un procédé comme défini dans l'une quelconque des revendications 11 à 15.
